# EUROPÄISCHE PATENTSCHRIFT

(11) **EP 0 731 801 B1**
(45) Veröffentlichungstag und Bekanntmachung des Hinweises auf die Patenterteilung: **24.10.2001**
(21) Anmeldenummer: 95901437.4
(22) Anmeldetag: 26.11.1994
(51) Int. Cl.: C07D 401/12, C07D 401/14, C07D 405/14, C07D 471/04, A61K 31/415, A61K 31/44, A61K 31/47, A61K 31/495

(54) **SUBSTITUIERTE AMINOALKYLAMINOPYRIDINE**
SUBSTITUTED AMINOALKYLAMINOPYRIDINES
AMINOALKYLAMINOPYRIDINES SUBSTITUEES

(30) Priorität: 01.12.1993 CH 358193
(43) Veröffentlichungstag der Anmeldung: 18.09.1996
(73) Patentinhaber: Byk Gulden Lomberg Chemische Fabrik GmbH, 78467 Konstanz (DE)
(72) Erfinder: KOHL, Bernhard, D-78465 Konstanz (DE); GRUNDLER, Gerhard, D-78464 Konstanz (DE); OPFERKUCH, Wolfgang, D-44801 Bochum (DE); SENN-BILFINGER, Jörg, D-78464 Konstanz (DE)
(86) Internationale Anmeldenummer: EP9403911
(87) Internationale Veröffentlichungsnummer: WO9515324

(56) Entgegenhaltungen:
- EP-A- 0 184 322
- EP-A- 0 567 643
- WO-A-93/24480

## Beschreibung

### Anwendungsgebiet der Erfindung

Die Erfindung betrifft Verbindungen, die in der pharmazeutischen Industrie als Wirkstoffe für die Herstellung von Arzneimitteln verwendet werden sollen.

### Bekannter technischer Hintergrund

In der internationalen Patentanmeldung WO92/12976 werden auf bestimmte Weise substituierte 2-(Pyridylmethylthio- bzw. -sulfinyl)-benzimidazole beschrieben, die gegen Helicobacter-Bakterien wirksam sein sollen und für die weiterhin offenbart ist, daß sie für die Verhütung und Behandlung einer ganzen Reihe von Erkrankungen des Magens geeignet sein sollen.

### Beschreibung der Erfindung

Gegenstand der Erfindung sind Verbindungen der Formel I (siehe beigefügtes Formelblatt), worin
R1 Wasserstoff bedeutet,
R2 Wasserstoff bedeutet,
R3 Halogen bedeutet,
R4 1-7C-Alkyl bedeutet,
A 1-7C-Alkylen bedeutet und
X N oder CH bedeutet,
   und worin
R5 1-7C-Alkyl, 3-8C-Cycloalkyl oder Ar-1-4C-alkyl und
R6 1-7C-Alkyl, 3-8C-Cycloalkyl oder Ar-1-4C-alkyl bedeutet, wobei

Ar Phenyl, Furyl oder durch R7, R8 und R9 substituiertes Phenyl bedeutet, oder worin
R5 und R6 gemeinsam und unter Einschluß des Stickstoffatoms, an das beide gebunden sind, einen unsubstituierten oder substituierten 6-Ring-Heterocyclus darstellen, der ausgewählt ist aus der Gruppe bestehend aus Piperidin, Piperazin 1,2,3,4-Tetrahydrochinolin und 1,2,3,4-Tetrahydroisochinolin,
   wobei
   - ein substituierter Piperidinorest substituiert ist mit einem oder zwei gleichen oder verschiedenen Substituenten ausgewählt aus der Gruppe bestehend aus
      1-4C-Alkyl,
      1-4C-Alkoxy,
      Phenyl,
      durch R7, R8 und R9 substituiertes Phenyl und
      Phenyl-1-4C-alkyl,
   - ein substituierter Piperazinorest in 4-Position substituiert ist mit einem Substituenten ausgewählt aus der Gruppe bestehend aus
      1-4C-Alkyl,
      1-4C-Alkoxycarbonyl,
      Phenyl,
      durch R7, R8 und R9 substituiertes Phenyl,
      Phenyl-1-4C-alkyl,
      durch R7, R8 und R9 im Phenylrest substituiertes Phenyl-1-4C-alkyl und Benzhydryl,
   - ein substituierter 1,2,3,4-Tetrahydrochinolinrest substituiert ist mit einem oder zwei gleichen oder verschiedenen Substituenten ausgewählt aus der Gruppe bestehend aus 1-4C-Alkyl und Halogen,
   - ein substituierter 1,2,3,4-Tetrahydroisochinolinrest am Benzoteil substituiert ist mit einem oder zwei Substituenten ausgewählt aus der Gruppe bestehend aus Hydroxy, 1-4C-Alkoxy und Di-1-4C-alkylamino, und wobei
R7 Wasserstoff, 1-4C-Alkyl, 1-4C-Alkoxy, Halogen oder Nitro,
R8 Wasserstoff, 1-4C-Alkyl, 1-4C-Alkoxy, Halogen oder Nitro,
   und
R9 Wasserstoff oder Trifluormethyl bedeutet,
   und die Salze dieser Verbindungen.

1-4C-Alkyl steht für geradkettige oder verzweigte Alkylreste mit 1 bis 4 Kohlenstoffatomen. Beispielsweise seien genannt der Butyl-, iso-Butyl-, sec.-Butyl-, tert.-Butyl-, Propyl-, Isopropyl-, Ethyl- und der Methylrest.

1-4C-Alkoxy steht für einen Rest, der neben dem Sauerstoffatom einen der vorstehend genannten 1-4C-Alkylreste enthält. Beispielsweise seien der Methoxy- und der Ethoxyrest genannt.

Halogen im Sinne der vorliegenden Erfindung ist Brom, Chlor und Fluor.

1-7C-Alkyl steht für geradkettige und verzweigte Alkylreste mit 1 bis 7 Kohlenstoffatomen. Beispielsweise seien genannt der Heptyl-, Hexyl-, Neopentyl-, Isopentyl-, Pentyl-, Butyl-, iso-Butyl-, sec.-Butyl-, tert.-Butyl-, Propyl-, Isopropyl-, Ethyl- und der Methylrest.

1-7C-Alkylen steht für geradkettige oder verzweigte 1-7C-Alkylenrest, beispielsweise den Methylen- (-CH₂-), Ethylen- (-CH₂-CH₂-), Trimethylen- (-CH₂-CH₂-CH₂-), Tetramethylen-(-CH₂-CH₂-CH₂-CH₂-), 1,2-Dimethylethylen- [-CH(CH₃)-], 1,1-Dimethylethylen-[-C(CH₃)₂-CH₂-], 2,2-Dimethylethylen- -CH₂-C(CH₃)₂-], Isopropyliden- [-C(CH₃)₂-], 1-Methylethylen- [-CH(CH₃)CH₂-], Pentamethylen- (-CH₂-CH₂-CH₂-CH₂-CH₂-), Hexamethylen- (-CH₂-CH₂-CH₂-CH₂-CH₂-CH₂-) und den Heptamethylenrest (-CH₂-CH₂-CH₂-CH₂-CH₂-CH₂-CH₂-).

3-8C-Cycloalkyl steht für den Cyclopropyl-, Cyclobutyl-, Cyclopentyl-, Cyclohexyl-, Cycloheptyl- und Cyclooctylrest.

Ar-1-4C-alkyl steht für einen der obengenannten, durch Ar substituierten 1-4C-Alkylrest. Beispielsweise seien der Phenethyl-, der Benzyl-, und der 2-Furylmethyl- (= Furfurylrest) genannt.

1-4C-Alkoxycarbonyl steht für einen Rest, der neben der Carbonylgruppe einen der vorstehend genannten 1-4C-Alkoxyreste enthält. Beispielsweise seien der Methoxycarbonyl- und der Ethoxycarbonylrest genannt.

Di-1-4C-alkylamino steht für einen Aminorest, der durch zwei gleiche oder verschiedene der vorstehend genannten 1-4C-Alkylreste substituiert ist. Beispielsweise seien der Dimethylamino-, der Diethylamino- und der Di-isopropylaminorest genannt.

Als beispielhafte, durch R7, R8 und R9 substituierte Phenylreste seien die Reste 3,4-Dimethoxy-, 2-Methoxy-, 2-Ethoxy-, 3-Methoxy-, 4-Methoxy-, 4-Fluor-, 4-Chlor, 2-Chlor-, 3-Chlor-, 3,4-Dichlor-, 3-Trifluormethyl-, 2-Trifluormethyl-, 2-Methyl-, 3-Methyl-, 4-Methyl-, 2,3-Dimethyl-, 2,4-Dimethyl-, 3,4-Dimethyl-, 2,5-Dimethyl-, 4-Nitro- und 2,6-Dinitro-4-trifluormethylphenyl genannt.

Als substituierte Piperidinoreste seien beispielsweise der 2-n-Propylpiperidino-, 5-Ethyl-2-methylpiperidino-, 4-n-Propylpiperidino-, 4-(3-Phenylpropyl)piperidino-, 4-Ethoxy-4-phenylpiperidino-, 4,4-Dimethylpiperidino-, 4-Phenyl-4-propyloxypiperidino-, 2,6-Dimethylpiperidino-, 2-Ethyl-2-methylpiperidino-, 2-Methylpiperidino-, 2,6-Dimethylpiperidino-, 2-Ethylpiperidino- und der 4-Benzylpiperidinorest genannt.

Als substituierte Piperazinoreste seien beispielsweise der 4-Methylpiperiazino-, 4-[2-(2-Trifluormethylphenyl)ethyl]piperazino-, 4-Phenylpiperazino-, 4-(2-Methylphenyl)piperazino-, 4-(2,3-Dimethylphenyl)piperazino-, 4-(2-Chlorphenyl)piperazino-, 4-(2-Methoxyphenyl)piperazino-, 4-(2-Ethoxyphenyl)piperazino-, 4-(3-Chlorphenyl)piperazino-, 4-(4-Fluorphenyl)piperazino-, 4-(4-Chlorphenyl)piperazino-, 4-(4-Methoxyphenyl)piperazino-, 4-(2,4-Dimethylphenyl)piperazino-, 4-(3,4-Dichlorphenyl)piperazino-, 4-(3,4-Dimethylphenyl)piperazino-, 4-Benzylpiperazino-, 4-Propylpiperazino-, 4-(3-Methylphenyl)piperazino-, 4-(3-Methoxyphenyl)piperazino-, 4-(4-Methylphenyl)piperazino-, 4-(2,5-Dimethylphenyl)piperazino-, 4-Benzhydrylpiperazino-, 4-n-Butylpiperazino-, 4-iso-Butylpiperazino-, 4-tert.-Butylpiperazino-, 4-(3-Trifluormethylphenyl)piperazino-, 4-(1-Phenylethyl)piperazino-, 4-(2-Phenylethyl)piperazino-, 4-(3,4-Dimethoxyphenyl)piperazino-, 4-lsopropylpiperazino-, 4-(2,6-Dinitro-4-trifluormethylphenyl)piperazino-, 4-(4-Nitrophenyl)piperazino- und der 4-Ethoxycarbonylpiperazinorest.

Als substituierte 1,2,3,4-Tetrahydrochinolinreste seien beispielsweise der 2-Ethoxycarbonyl-1,2,3,4-tetrahydro-1-chinolinyl-, 2-Methyl-1,2,3,4-tetrahydro-1-chinolinyl-, 6-Methyl-1,2,3,4-tetrahydro-1-chinolinyl-, 6-Fluor-2-methyl-1,2,3,4-tetrahydro-1-chinolinyl-, 4-Methyl-1,2,3,4-tetrahydro-1-chinolinyl- und der 2-Fluor-6-methyl--1,2,3,4-tetrahydro-1-chinolinylrest genannt.

Als substituierte 1,2,3,4-Tetrahydroisochinolinreste seien beispielsweise der 6,7-Dimethoxy-1,2,3,4-tetrahydro-2-isochinolinyl- und der 6,7-Dihydroxy-1,2,3,4-tetrahydro-2-isochinolinylrest genannt.

Als Salze kommen für Verbindungen der Formel I alle Säureadditionssalze in Betracht. Besonders erwähnt seien die pharmakologisch verträglichen Salze der in der Galenik üblicherweise verwendeten anorganischen und organischen Säuren. Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt. Als solche eignen sich wasserlösliche und wasserunlösliche Säureadditionssalze mit Säuren wie beispielsweise Salzsäure, Bromwasserstoffsäure, Phosphorsäure, Salpetersäure, Schwefelsäure, Essigsäure, Zitronensäure, D-Gluconsäure, Benzoesäure, 2-(4-Hydroxybenzoyl)-benzoesäure, Buttersäure, Sulfosalicylsäure, Maleinsäure, Laurinsäure, Äpfelsäure, Fumarsäure, Bernsteinsäure, Oxalsäure, Weinsäure, Embonsäure, Stearinsäure, Toluolsulfonsäure, Methansulfonsäure oder 3-Hydroxy-2-naphtoesäure, wobei die Säuren bei der Salzherstellung - je nachdem, ob es sich um eine ein- oder mehrbasige Säure handelt und je nachdem, welches Salz gewünscht wird - im äquimolaren oder einem davon abweichenden Mengenverhältnis eingesetzt werden.

Besonders hervorzuheben sind solche Verbindungen der Formel I, in denen R1 Wasserstoff, R2 Wasserstoff, R3 Chlor, R4 1-4C-Alkyl, A Ethylen oder Propylen und X CH bedeutet, und ihre Salze.

Eine Ausgestaltung innerhalb der hervorzuhebenden Verbindungen sind solche Verbindungen der Formel I, in denen R1 Wasserstoff, R2 Wasserstoff, R3 Halogen, R4 1-4C-Alkyl, A 2-4C-Alkylen und X N oder CH bedeutet, und worin R5 1-4C-Alkyl oder Ar-1-4C-alkyl und R6 Ar-1-4C-alkyl bedeutet, wobei Ar Phenyl, Furyl oder durch R7, R8 und R9 substituiertes Phenyl bedeutet, oder worin R5 und R6 gemeinsam und unter Einschluß des Stickstoffatoms, an das beide gebunden sind, einen unsubstituierten. oder substituierten 6-Ring-Heterocyclus darstellen, der ausgewählt ist aus der Gruppe bestehend aus Piperidin, Piperazin, 1,2,3,4-Tetrahydrochinolin und 1,2,3,4-Tetrahydroisochinolin wobei
- ein substituierter Piperidinorest substituiert ist mit einem oder zwei gleichen oder verschiedenen Substituenten ausgewählt aus der Gruppe bestehend aus 1-4C-Alkyl, 1-4C-Alkoxy, Phenyl, durch R7, R8 und R9 substituiertes Phenyl und Phenyl-1-4C-alkyl,
- ein substituierter Piperazinorest in 4-Position substituiert ist mit einem Substituenten ausgewählt aus der Gruppe bestehend aus 1-4C-Alkyl, 1-4C-Alkoxycarbonyl, Phenyl, durch R7, R8 und R9 substituiertes Phenyl, Phenyl-1-4C-alkyl, durch R7, R8 und R9 im Phenylrest substituiertes Phenyl-1-4C-alkyl und Benzhydryl
- ein substituierter 1,2,3,4-Tetrahydrochinolinrest substituiert ist mit einem oder zwei gleichen oder verschiedenen Substituenten ausgewählt aus der Gruppe bestehend aus 1-4C-Alkyl und Halogen,
- ein substituierter 1,2,3,4-Tetrahydroisochinolinrest am Benzoteil substituiert ist mit einem oder zwei Substituenten ausgewählt aus der Gruppe bestehend aus Hydroxy, 1-4C-Alkoxy und Di-1-4C-alkylamino, und wobei

- R7: Wasserstoff, 1-4C-Alkyl, 1-4C-Alkoxy, Halogen oder Nitro,
- R8: Wasserstoff, 1-4C-Alkyl, 1-4C-Alkoxy, Halogen oder Nitro, und
- R9: Wasserstoff oder Trifluormethyl bedeutet, und die Salze dieser Verbindungen.

Eine Ausgestaltung innerhalb der besonders hervorzuhebenden Verbindungen sind solche Verbindungen der Formel I, in denen R1 Wasserstoff, R2 Wasserstoff, R3 Chlor, R4 1-4C-Alkyl, A Ethylen oder Propylen und X CH bedeutet, und worin R5 1-4C-Alkyl oder Benzyl und R6 Ar-1-4C-alkyl bedeutet, wobei Ar Phenyl oder Furyl bedeutet, oder worin R5 und R6 gemeinsam und unter Einschluß des Stickstoffatoms, an das beide gebunden sind, einen unsubstituierten oder substituierten 6-Ring-Heterocyclus darstellen, der ausgewählt ist aus der Gruppe bestehend aus Piperidin, Piperazin und 1,2,3,4-Tetrahydroisochinolin wobei
- ein substituierter Piperidinorest substituiert ist mit einem Substituenten ausgewählt aus der Gruppe bestehend aus Phenyl und Benzyl,
- ein substituierter Piperazinorest in 4-Position substituiert ist mit einem Substituenten ausgewählt aus der Gruppe bestehend aus Phenyl, durch R7, R8 und R9 substituiertes Phenyl und Benzyl und
- ein substituierter 1,2,3,4-Tetrahydroisochinolinrest am Benzoteil substituiert ist mit einem oder zwei 1-4C-Alkoxy-Substituenten, und wobei

- R7: Wasserstoff oder 1-4C-Alkoxy,
- R8: Wasserstoff und
- R9: Wasserstoff bedeutet, und die Salze dieser Verbindungen.

Ein weiterer Gegenstand der Erfindung ist ein Verfahren zur Herstellung der Verbindungen der Formel I, worin R1, R2, R3, R4, R5, R6, X und A die oben angegebenen Bedeutungen haben, und ihrer Salze.

Das Verfahren ist dadurch gekennzeichnet, daß man
a) Mercaptobenzimidazole der Formel II (siehe beigefügtes Formelblatt), worin R1, R2 und X die oben angegebenen Bedeutungen haben, mit Picolinderivaten III (siehe beigefügtes Formelblatt), worin R3, R4, R5, R6 und A die oben angegebenen Bedeutungen haben und Y eine geeignete Abgangsgruppe darstellt, umsetzt oder daß man
b) Verbindungen der Formel IV (siehe beigefügtes Formelblatt), worin R1, R2, R3, R4, X und A die oben angegebenen Bedeutungen haben und Z eine geeignete Abgangsgruppe darstellt, mit Aminen H-N(R5)R6 umsetzt und
daß man erhaltene Verbindungen gewünschtenfalls anschließend in die Salze überführt und/oder daß man erhaltene Salze gewünschtenfalls anschließend in die freien Verbindungen überführt.

Bei der vorstehend aufgeführten Umsetzung können die Ausgangsverbindungen als solche oder gegebenenfalls in Form ihrer Salze eingesetzt werden.

Als geeignete Abgangsgruppen Y bzw. Z seien beispielsweise Halogenatome, insbesondere Chlor, oder durch Veresterung (z.B. mit p-Toluolsulfonsäure) aktivierte Hydroxylgruppen genannt.

Die Umsetzung von II mit III erfolgt in geeigneten, vorzugsweise polaren protischen oder aprotischen Lösungsmitteln (wie Methanol, Ethanol, Isopropanol, Dimethylsulfoxid, Aceton, Dimethylformamid oder Acetonitril) unter Zusatz oder unter Ausschluß von Wasser. Sie wird beispielsweise in Gegenwart eines Protonenakzeptors durchgeführt. Als solche eignen sich Alkalimetallhydroxide, wie Natriumhydroxid, Alkalimetallcarbonate, wie Kaliumcarbonat, oder tertiäre Amine, wie Pyridin, Triethylamin oder Ethyldiisopropylamin. Alternativ kann die Umsetzung auch ohne Protonenakzeptor durchgeführt werden, wobei - je nach Art der Ausgangsverbindungen - gegebenenfalls zunächst die Säureadditionssalze in besonders reiner Form abgetrennt werden können. Die Reaktionstemperatur kann zwischen 0° und 150°C liegen, wobei in Gegenwart von. Protonenakzeptoren Temperaturen zwischen 20° und 80°C und ohne Protonenakzeptoren zwischen 60° und 120°C - insbesondere die Siedetemperatur der verwendeten Lösungsmittel - bevorzugt sind. Die Reaktionszeiten liegen zwischen 0,5 und 30 Stunden.

Die Umsetzung der Verbindungen IV mit den Aminen H-N(R5)R6 erfolgt auf ähnliche Weise wie die Umsetzung der Verbindungen II mit den Verbindungen III bzw. alternativ bevorzugt ohne zusätzliches Lösungsmittel unter Verwendung eines Überschusses an Amin als Protonenakzeptor und gleichzeitig Lösungsmittel. Die Reaktionstemperatur liegt in diesem Fall zwischen 60° und 180°C, bevorzugt zwischen 80° und 160°C.

Die Verbindungen II sind z.B. aus DE 34 04 610 oder EP 134 400 bekannt. Die Verbindungen III können beispielsweise so wie in den nachfolgenden Beispielen beschrieben bzw. in Analogie zu EP 184 322 hergestellt werden.

Die Verbindungen der Formel IV können beispielsweise so, wie in den nachfolgenden Beispielen beschrieben, aus bekannten oder in analoger Weise erhältlichen Ausgangsverbindungen hergestellt werden.

Die folgenden Beispiele erläutern die Erfindung näher, ohne sie einzuschränken. Die erfindungsgemäßen Verbindungen und die Ausgangsverbindungen können auf analoge Weise wie in den Beispielen beschrieben hergestellt werden.

### Beispiele

### Endprodukte

### 1. 2-{3-Chlor-4-{N-[2-(N-benzyl-N-ethylamino)-ethyl]-N-methylamino}-2-pyridyl}-methylthio-1H-benzimidazol-trihydrochlorid

2-{3-Chlor-4-[N-(2-chlorethyl)-N-methylamino]-2-pyridyl}-methylthio-1H-benzimidazol (500 mg/1,24 mmol) werden in N-Ethylbenzylamin (15 ml) 4,5 h bei 140°C erhitzt. Danach wird das überschüssige N-Ethylbenzylamin im Hochvakuum abdestilliert und der Rückstand an Kieselgel chromatographiert (Dichlormethan/Methanol 97/3-Mischung, die 1 ml konz. NH₃ x aq./L enthält). Die gesammelten, reinen Fraktionen werden gemeinsam im Vakuum eingeengt, in wenig Methanol gelöst und mit gesättigter, etherischer Salzsäure (1 ml) und Diisopropylether versetzt und der hierbei ausfallende Feststoff abfiltriert und im Vakuum getrocknet. Ausbeute: 200 mg (28 %) der Titelverbindung als farbloser Feststoff vom Schmp. > 180°C (Zers.).

### 2. 2-{3-Chlor-4-{N-[2-(1,2,3,4-tetrahydro-isochinolin-2-yl)-ethyl]-N-methylamino}-2-pyridyl}-methylthio-1 H-benzimidazol-trihydrochlorid

2-{3-Chlor-4-[N-2-chlorethyl)-N-methylamino]-2-pyridyl}-methylthio-1H-benz-imidazol (500 mg) werden in 1,2,3,4-Tetrahydro-isochinolin (10 ml) 2,5 h bei 100°C erhitzt. Das überschüssige Amin wird im Hochvakuum abdestilliert und der verbleibende ölige Rückstand an Kieselgel chromatographiert (Petrolether/Ethylacetat/Methanol 65/30/5-Mischung, die 1 ml konz. NH₃ x aq./L enthält). Die gesammelten, reinen Fraktionen werden gemeinsam im Vakuum eingeengt, in wenig Methanol gelöst (5 ml), mit etherischer Salzsäure und danach mit wenig Diisopropylether versetzt. Der hierbei ausfallende Feststoff wird abfiltriert und im Hochvakuum getrocknet. Ausbeute: 460 mg (65 %) der Titelverbindung als farbloser Feststoff vom Schmp. > 240°C (Zers.).

### 3. 2-{3-Chlor-4-{N-[2-(1,2,3,4-tetrahydro-isochinolin-2-yl)-ethyl]-N-methylamino}-2-pyridyl}-methylthio-1H-benzimidazol

2-{3-Chlor-4-{N-[2-(4-1,2,3,4-tetrahydro-isochinolin-2-yl)-ethyl]-N-methylamino}-2-pyridyl}-methylthio-1H-benzimidazol-trihydrochlorid (0,1 g) wird in Wasser gelöst (7 ml) und mit einer gesättigten Lösung von wäßrigem Natriumhydrogencarbonat (1 ml) versetzt. Der hierbei ausfallende, farblose Niederschlag wird abfiltriert, mit destilliertem Wasser gewaschen und bei 60°C im Hochvakuum getrocknet. Ausbeute: 70 mg (87 %) der Titelverbindung vom Schmp. > 88°C (Zers.).

### 4. 2-{3-Methyl-4-{N-{2-[N-(2-furfuryl)-N-methylamino]-ethyl}-N-methylamino}-2-pyridyl}-methylthio-1H-benzimidazol-trihydrochlorid

Analog Beispiel 1 werden 1,35 g (64 %) der Titelverbindung vom Schmp. 212°C (Zers.) durch Umsetzung von 2-{3-Chlor-4-[N-2-chlorethyl)-N-methylamino]-2-pyridyl}-methylthio-1 H-benzimidazol (1,5 g) mit N-Furfurylmethylamin (2 ml) nach 4-stündigem Erhitzen bei 100°C erhalten.

### 5. 2-{3-Chlor-4-{N-[2-(1,2,3,4-tetrahydro-isochinolin-2-yl)-ethyl]-N-methylamino}-2-pyridyl}-methylthio-1H-imidazo[5,4-b]pyridin

Nach der in Beispiel 2 angegebenen Arbeitsweise erhält man, ausgehend von 2-{3-Chlor-4-[N-(2-chlorethyl)-N-methylamino]-2-pyridyl}-methylthio-1 H-imidazo[5,4-b]pyridin und 1,2,3,4-Tetrahydro-isochinolin nach Chromatographie an Kieselgel (Ethylacetat/Methanol 4/1) die Titelverbindung als farbloses Pulver vom Schmp. 128-129°C (52 %).

### 6. 2-{3-Chlor-4-{N-[2-(N-benzyl-N-methylamino)-ethyl]-N-methylamino}-2-pyridyl}-methylthio-1H-benzimidazol-trihydrochlorid

Nach der in Beispiel 1 angegebenen Arbeitsweise erhält man die Titelverbindung als amorphes Pulver vom Schmp. 237-240°C (Zers.) durch Umsetzung von 2-{3-Chlor-4-[N-(2-chlorethyl)-N-methylamino]-2-pyridyl}-methylthio-1 H-benzimidazol mit N-Methylbenzylamin nach Reinigung an Kieselgel und anschließender Überführung in das Trihydrochlorid.

### 7. 2-{3-Chlor-4-{N-[2-(N,N-dibenzylamino)-ethyl]-N-methylamino}-2-pyridyl}-methylthio-1H-benzimidazol-trihydrochlorid

Nach der in Beispiel 2 angegebenen Arbeitsweise erhält man die Titelverbindung als farbloses, amorphes Pulver vom Schmp. Öl durch Umsetzung von 2-{3-Chlor-4-[N-(2-chlorethyl)-N-methylamino]-2-pyridyl}-methylthio-1 H-benzimi-dazol mit Dibenzylamin nach Chromatographie an Kieselgel (Petrolether/Ethylacetat 1/1).

### 8. 2-{3-Chlor-4-{N-[2-(N,N-diethylamino)-ethyl]-N-methylamino}-2-pyridyl}-methylthio-1H-benzimidazol-trihydrochlorid

Nach der in Beispiel 1 angegebenen Arbeitsweise erhält man die Titelverbindung als amorphes Pulver vom Schmp. 245,7°C (Zers.) durch Umsetzung von 2-{3-Chlor-4-[N-2-chlorethyl)-N-methylamino]-2-pyridyl}-methylthio-1H-benz-imidazol mit Diethylamin nach Chromatographie an Kieselgel (Ethylacetat/-Methanol 4/1) und anschließender Überführung in das Trihydrochlorid.

### 9. 2-{3-Chlor-4-{N-[2-(N-methyl-N-phenethylamino)-ethyl]-N-methylamino}-2-pyridyl}-methylthio-1H-benzimidazol-trihydrochlorid

Nach der in Beispiel 1 angegebenen Arbeitsweise erhält man die Titelverbindung als amorphes Pulver vom Schmp. 239-241°C (Zers.) durch Umsetzung von 2-{3-Chlor-4-[N-(2-chlorethyl)-N-methylamino]-2-pyridyl}-methylthio-1H-benzimidazol mit N-Methyl-phenethylamin nach Reinigung an Kieselgel (Ethylacetat/Methanol 9/1) und anschließender Überführung in das Trihydrochlorid.

### 10. 2-{3-Chlor-4-{N-[2-(N-furfuryl-N-methylamino)-ethyl]-N-methylamino}-2-pyridyl}-methylthio-1H-benzimidazol-trihydrochlorid

Nach der in Beispiel 1 angegebenen Arbeitsweise erhält man die Titelverbindung als bräunliches, amorphes Pulver vom Schmp. 239-241°C (Zers.) durch Umsetzung von 2-{3-Chlor-4-[N-(2-chlorethyl)-N-methylamino]-2-pyridyl}-me-thylthio-1H-benzimidazol mit N-Furfuryl-methylamin nach Chromatographie an Kieselgel (Ethylacetat/Methanol 9/1) und anschließender Überführung in das Trihydrochlorid.

### 11. 2-{3-Chlor-4-{N-[2-(4-phenyl-piperidino)-ethyl]-N-methylamino}-2-pyridyl)-methylthio-1H-benzimidazol

Nach der in Beispiel 2 angegebenen Arbeitsweise erhält man die Titelverbindung als amorphes, gelbliches Pulver vom Schmp. 55-65°C durch Umsetzung von 2--{3-Chlor-4-[N-(2-chlorethyl)-N-methylamino]-2-pyridyl}-methylthio-1H-benzimidazol mit 4-Phenylpiperidin nach Reinigung an Kieselgel (Ethylacetat/Methanol 3/1).

### 12. 2-{3-Chlor-4-{N-[2-(4-benzyl-piperidino)-ethyl]-N-methylamino}-2-pyridyl}-methylthio-1H-benzimidazol

Nach der in Beispiel 2 angegebenen Arbeitsweise erhält man die Titelverbindung als zähes, gelbliches Öl durch Umsetzung von 2-{3-Chlor-4-[N-(2-chlorethyl)-N-methylamino]-2-pyridyl}-methylthio-1 H-benzimidazol mit 4-Benzylpiperidin nach Reinigung an Kieselgel (Ethylacetat/Methanol 4/1).

### 13. 2-{3-Chlor-4-[N-(2-piperidinoethyl)-N-methylamino]-2-pyridyl}-methylthio-1H-benzimidazol

Nach der in Beispiel 2 angegebenen Arbeitsweise erhält man die Titelverbindung als zähes, bräunliches Öl durch Umsetzung von 2-{3-Chlor-4-[N-(2-chlorethyl)-N-methylamino]-2-pyridyl}-methylthio-1H-benzimidazol mit Piperidin nach Chromatographie an Kieselgel (Ethylacetat/Methanol 4/1).

### 14. 2-{3-Chlor-4-{N-[2-(6,7-dimethoxy-1,2,3,4-tetrahydro-isochinolin-2-yl)-ethyl]-N-methylamino}-2-pyridyl}-methylthio-1H-benzimidazol

Nach der in Beispiel 2 angegebenen Arbeitsweise erhält man die Titelverbindung als zähes, gelbliches Öl durch Umsetzung von 2-{3-Chlor-4-[N-(2-chlorethyl)-N-methylamino]-2-pyridyl}-methylthio-1 H-benzimidazol mit 6,7-Dimethoxy-1,2,3,4-tetrahydro-isochinolin nach Chromatographie an Kieselgel (Ethylacetat/Methanol 4/1).

### 15. 2-{3-Chlor-4-{N-[2-(4-phenylpiperazino)-ethyl]-N-methylamino}-2-pyridyl}-methylthio-1H-benzimidazol-hydrochlorid

Nach der in Beispiel 1 angegebenen Arbeitsweise erhält man die Titelverbindung als amorphes Pulver vom Schmp. 212-215°C (Zers.) durch Umsetzung von 2-{3-Chlor-4-[N-(2-chlorethyl)-N-methylamino]-2-pyridyl}-methylthio-1 H-benzimidazol mit 4-Phenylpiperazin nach Chromatographie an Kieselgel (Ethylacetat/Methanol 4/1) und anschließender Überführung in das Hydrochlorid.

### 16. 2-{3-Chlor-4-{N-[2-(4-benzylpiperazino)-ethyl]-N-methylamino}-2-pyridyl}-methylthio-1H-benzimidazol-trihydrochlorid

Nach der in Beispiel 1 angegebenen Arbeitsweise erhält man die Titelverbindung als amorphes Pulver vom Schmp. 227-230°C (Zers.) durch Umsetzung von 2-{3-Chlor-4-[N-(2-chlorethyl)-N-methylamino]-2-pyridyl}-methylthio-1H-benzimidazol mit 4-Benzylpiperazin nach Chromatographie an Kieselgel (Ethylacetat/Methanol 4/1) und anschließender Überführung in das Trihydrochlorid.

### 17. 2-{3-Chlor-4-{N-[2-(4-(2-methoxyphenyl)-piperazino)-ethyl]-N-methylamino}-2-pyridyl}-methylthio-1H-benzimidazol-trihydrochlorid

Nach der in Beispiel 1 angegebenen Arbeitsweise erhält man die Titelverbindung als zähes Öl durch Umsetzung von 2-{3-Chlor-4-[N-(2-chlorethyl)-N-methylamino]-2-pyridyl}-methylthio-1 H-benzimidazol mit 4-(2-Methoxyphenyl)-piperazin nach Reinigung an Kieselgel (Ethylacetat/Methanol 9/1).

### 18. 2-{3-Chlor-4-{N-[3-(1,2,3,4-tetrahydro-isochinolin-2-yl)-propyl]-N-methylamino}-2-pyridyl}-methylthio-1H-benzimidazol-trihydrochlorid

Die Titelverbindung vom Schmp. 239-240°C (Zers.) wird analog Beispiel 2 durch Umsetzung von 2-{3-Chlor-4-[N-(3-chlorpropyl)-N-methylamino]-2-pyridyl}-methylthio-1H-benzimidazol mit 1,2,3,4-Tetrahydro-isochinolin und Überführung in das Trihydrochlorid erhalten.

### 19. 2-{3-Chlor-4-{N-[3-(N-benzyl-N-methylamino)-propyl]-N-methylamino}-2-pyridyl}-methylthio-1H-benzimidazol-trihydrochlorid

Nach der in Beispiel 1 angegebenen Arbeitsweise erhält man die Titelverbindung als amorphes Pulver vom Schmp. 228-230°C (Zers.) durch Umsetzung von 2-{3-Chlor-4-[N-(3-chlorpropyl)-N-methylamino]-2-pyridyl}-methylthio-1H-benzimidazol mit N-Methylbenzylamin nach Reinigung an Kieselgel und anschließender Überführung in das Trihydrochlorid.

### 20. 2-{3-Chlor-4-{N-[3-(6,7-dimethoxy-1,2,3,4-tetrahydro-isochinolin-2-yl)-propyl]-N-methylamino}-2-pyridyl}-methylthio-1H-benzimidazol-trihydrochlorid

Nach der in Beispiel 1 angegebenen Arbeitsweise erhält man die Titelverbindung als zähes, gelbliches Öl durch Umsetzung von 2-{3-Chlor-4-[N-(3-chlorpropyl)-N-methylamino]-2-pyridyl}-methylthio-1 H-benzimidazol mit 6,7-Dimethoxy-1,2,3,4-tetrahydro-isochinolin nach Chromatogaphie an Kieselgel (Petrolether/Ethylacetat/Methanol 2/5/1).

### 21. 2-{3-Chlor-4-{N-[3-(4-benzyl-piperidino)-propyl]-N-methylamino}-2-pyridyl}-methylthio-1H-benzimidazol-trihydrochlorid

Nach der in Beispiel 1 angegebenen Arbeitsweise erhält man die Titelverbindung als zähes, gelbliches Öl durch Umsetzung von 2-{3-Chlor-4-[N-(3-chlorpropyl)-N-methylamino]-2-pyridyl}-methylthio-1 H-benzimidazol mit 4-Benzylpiperidin nach Reinigung an Kieselgel (Ethylacetat/Methanol 4/1) und Überführung in das Trihydrochlorid.

### Vorstufen

### A. 2-{3-Chlor-4-fN-(2-chlorethyl)-N-methylamino]-2-pyridyl}-methylthio-1H-benzimidazol-dihydrochlorid

1) 3-Chlor-4-[N-(2-hydroxyethyl)-N-methylamino]-2-hydroxymethylpyridin
   Eine Mischung aus 3,4-Dichlor-2-hydroxymethylpyridin (J.Med.Chem. 1989, 32, 1970) (2,5 g) in 2-Methylaminoethanol (30 ml) wird in einem Stahlautoklaven 2,5 h bei 160°C erwärmt, das überschüssige Amin im Hochvakuum abgezogen und der verbleibende Rückstand an Kieselgel chromatographiert (Dichlormethan/Methanol 95/5). Ausbeute: 2,3 g als gelbliches Öl.
2) 3-Chlor-4-[N-(2-chlorethyl)-N-methylamino]-2-chlormethylpyridin-hydro-chlorid
   Eine Lösung von 3-Chlor-4-[N-(2-hydroxyethyl)-N-methylamino]-2-hydroxymethylpyridin (2,3 g) in Dichlormethan (30 ml) wird bei 0°C tropfenweise mit einer Lösung von Thionylchlorid (4 ml) in Dichlormethan (20 ml) versetzt. Anschließend läßt man die Temperatur auf 20°C steigen (20 Min) und hält danach die Temperatur 30 Min bei 40°C. Nach Abziehen des Lösungsmittels im Vakuum wird der verbleibende Rückstand an Kieselgel chromatographiert (Petrolether/Ethylacetat 7/3 Mischung, die 1 ml konz. NH₃ x aq/L enthält). Ausbeute: 2,6 g.
3) Eine Mischung aus 2-Mercapto-1H-benzimidazol (1,8 g) und 3-Chlor-4-[N-(2-chlorethyl)-N-methylamino]-2-chlormethylpyridin-hydrochlorid (1,1 g) in Isopropanol (40 ml) wird 1,5 h am Sieden gehalten, das Lösungsmittel im Vakuum bis zu einem Volumen von 20 ml abgezogen und diese Lösung mit Diisopropylether (20 ml) versetzt. Die nach einiger Zeit ausfallenden Kristalle werden abfiltriert und im Vakuum getrocknet. Ausbeute: 1,2 g der Titelverbindung vom Schmp. 202°C (Zers.).

In analoger Weise wird
2-{3-Chlor-4-[N-(3-chlorpropyl)-N-methylamino]-2-pyridyl}-methylthio-1H-benzimidazol-dihydrochlorid erhalten.

### B. 2-{3-Chlor-4-[N-(2-chlorethyl)-N-methylamino]-2-pyridyl}-methylthio-1H-imidazo[5,4-b]pyridin

Eine Mischung von 3-Chlor-4-[N-(2-chlorethyl)-N-methylamino]-2-chlor-methylpyridin-hydrochlorid (0,96 g) und 2-Mercapto-1H-imidazo[5,4-b]pyridin (0,5 g) in Isopropanol (25 ml) wird 4 h bei 90°C erwärmt und das Reaktionsgemisch anschließend auf 0°C gekühlt. Die ausgefallenen Kristalle werden abfiltriert und mit wenig kaltem Isopropanol gewaschen. Der Filterkuchen wird in Wasser gelöst (30 ml), die Lösung mit gesättigter, wäßriger Natriumhydrogencarbonat-Lösung (20 ml) versetzt und mit Dichlormethan extrahiert. Die gesammelten Extrakte werden im Vakuum bis zur Trockene eingeengt und der verbleibende, kristalline Rückstand im Hochvakuum getrocknet. Ausbeute: 0,68 g der Titelverbindung vom Schmp. 184-185°C.

### Gewerbliche Anwendbarkeit

Die ausgezeichnete Wirksamkeit von Verbindungen der Formel I und ihren Salzen gegen Helicobacter-Bakterien gestattet ihren Einsatz in der Humanmedizin als Wirkstoffe für die Behandlung von Krankheiten, die auf Helicobacter-Bakterien beruhen.

Ein weiterer Gegenstand der Erfindung ist daher ein Verfahren zur Behandlung von Säugern, insbesondere Menschen, die an Krankheiten erkrankt sind, die auf Helicobacter-Bakterien beruhen. Das Verfahren ist dadurch gekennzeichnet, daß man dem erkrankten Individuum eine therapeutisch wirksame und pharmakologisch verträgliche Menge einer oder mehrerer Verbindungen der Formel I und/oder ihrer pharmakologisch verträglichen Salze verabreicht.

Gegenstand der Erfindung sind außerdem die Verbindungen der Formel I und ihre pharmakologisch verträglichen Salze zur Anwendung bei der Behandlung von Krankheiten, die auf Helicobacter-Bakterien beruhen.

Ebenso umfaßt die Erfindung die Verwendung von Verbindungen der Formel I und ihren pharmakologisch verträglichen Salzen bei der Herstellung von Arzneimitteln, die zur Bekämpfung solcher Krankheiten eingesetzt werden, die auf Helicobacter-Bakterien beruhen.

Ein weiterer Gegenstand der Erfindung sind Arzneimittel zur Bekämpfung von Helicobacter-Bakterien, die eine oder mehrere Verbindungen der allgemeinen Formel I und/oder ihre pharmakologisch verträglichen Salze enthalten.

Von den Helicobacter-Stämmen, gegenüber denen sich die Verbindungen der Formel I als wirksam erweisen, sei insbesondere der Stamm Helicobacter pylori erwähnt.

Die Arzneimittel werden nach an sich bekannten, dem Fachmann geläufigen Verfahren hergestellt. Als Arzneimittel werden die pharmakologisch wirksamen Verbindungen der Formel I und ihre Salze (=Wirkstoffe) entweder als solche, oder vorzugsweise in Kombination mit geeigneten pharmazeutischen Hilfsstoffen z.B. in Form von Tabletten, Dragees, Kapseln, Emulsionen, Suspensionen, Gelen oder Lösungen eingesetzt, wobei der Wirkstoffgehalt vorteilhafterweise zwischen 0,1 und 95 % beträgt.

Welche Hilfsstoffe für die gewünschten Arzneimittelformulierungen geeignet sind, ist dem Fachmann aufgrund seines Fachwissens geläufig. Neben Lösemitteln, Gelbildnern, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Farbstoffe oder Permeationspromotoren und Komplexbildner (z.B. Cyclodextrine) verwendet werden.

Die Wirkstoffe können beispielsweise parenteral (z.B. intravenös) oder insbesondere oral appliziert werden.

Im allgemeinen werden in der Humanmedizin die Wirkstoffe in einer Tagesdosis von etwa 0,2 bis 50, vorzugsweise 1 bis 30 mg/kg Körpergewicht, gegebenenfalls in Form mehrerer, vorzugsweise 2 bis 6 Einzelgaben zur Erzielung des gewünschten Ergebnisses verabreicht.

In diesem Zusammenhang ist als erfindungswesentlicher Aspekt besonders zu erwähnen, daß sich die Verbindungen der Formel I gegenüber Helicobacter-Bakterien bereits bei Verabfolgung solcher Dosen als wirksam erweisen, die unterhalb der Dosen liegen, die zur Erzielung einer - therapeutischen Zwecken genügenden - Magensäuresekretionshemmung eingesetzt werden müßten.

### Biologische Untersuchungen

Die Verbindungen der Formel I wurden bezüglich ihrer Wirksamkeit gegenüber Helicobacter pylori in Anlehnung an die von Tomoyuki Iwahi et al. (Antimicrobial Agents and Chemotherapy, 1991, 490-496) beschriebene Methodik unter Verwendung von Columbia-agar (Oxoid) und bei einer Wachstumsperiode von 4 Tagen untersucht. Für die untersuchten Verbindungen ergaben sich hierbei die in der nachfolgenden Tabelle aufgeführten MIC-Werte (die angegebenen Nummern der Verbindungen stimmen mit den Verbindungsnummern in der Beschreibung überein).

**TABELLE**

| Verbindung Nr. | MIC-Wert (µg/ml) |
|---|---|
| 1 | <1 |
| 2 | <1 |
| 3 | <1 |
| 4 | <1 |
| 5 | <1 |
| 6 | <1 |
| 9 | <1 |
| 10 | <1 |
| 11 | <1 |
| 12 | <1 |
| 13 | <1 |
| 14 | <1 |
| 15 | <1 |
| 16 | <1 |
| 17 | <1 |
| 18 | <1 |
| 19 | <1 |
| 20 | <1 |
| 21 | <1 |

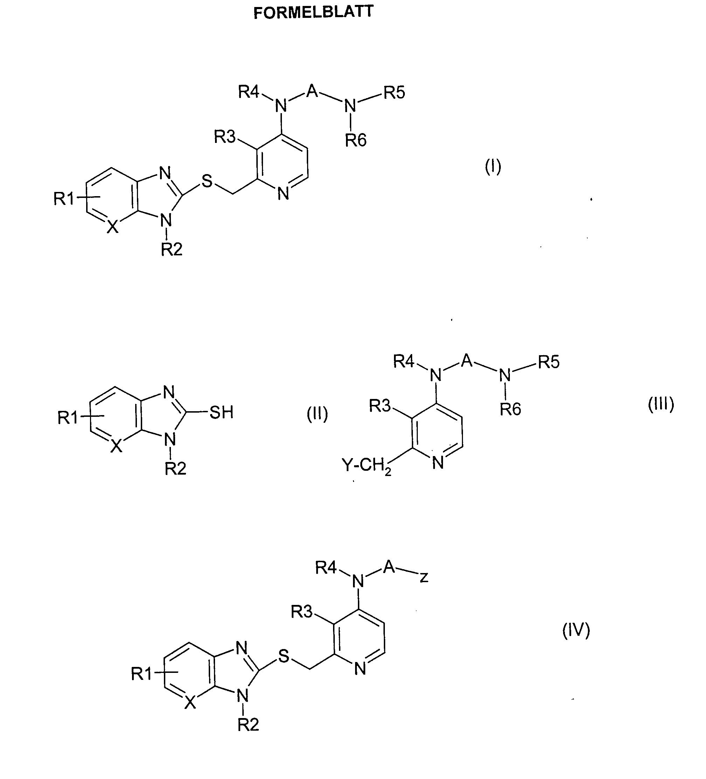

## Patentansprüche

1. Verbindungen der Formel I 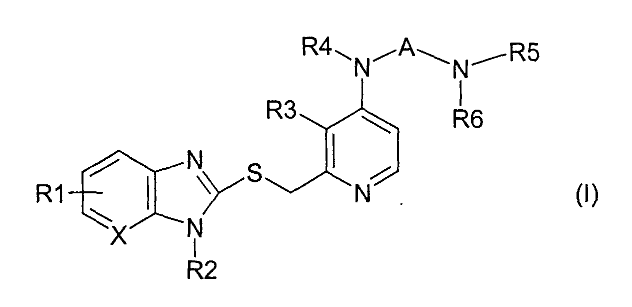 worin
R1 Wasserstoff bedeutet,
R2 Wasserstoff bedeutet,
R3 Halogen bedeutet,
R4 1-7C-Alkyl bedeutet,
A 1-7C-Alkylen bedeutet und
x N oder CH bedeutet
und worin
R5 1-7C-Alkyl, 3-8C-Cycloalkyl oder Ar-1-4C-alkyl und
R6 1-7C-Alkyl, 3-8C-Cycloalkyl oder Ar-1-4C-alkyl bedeutet,
wobei
Ar Phenyl, Furyl oder durch R7, R8 und R9 substituiertes Phenyl bedeutet, oder worin
R5 und R6 gemeinsam und unter Einschluß des Stickstoffatoms, an das beide gebunden sind, einen unsubstituierten oder substituierten 6-Ring-Heterocyclus darstellen, der ausgewählt ist aus der Gruppe bestehend aus Piperidin, Piperazin, 1,2,3,4-Tetrahydrochinolin und 1,2,3,4-Tetrahydroisochinolin, wobei
- ein substituierter Piperidinorest substituiert ist mit einem oder zwei gleichen oder verschiedenen Substituenten ausgewählt aus der Gruppe bestehend aus 1-4C-Alkyl, 1-4C-Alkoxy, Phenyl, durch R7, R8 und R9 substituiertes Phenyl und Phenyl-1-4C-alkyl,
- ein substituierter Piperazinorest in 4-Position substituiert ist mit einem Substituenten ausgewählt aus der Gruppe bestehend aus 1-4C-Alkyl, 1-4C-Alkoxycarbonyl, Phenyl, durch R7, R8 und R9 substituiertes Phenyl, Phenyl-1-4C-alkyl, durch R7, R8 und R9 im Phenylrest substituiertes Phenyl-1-4C-alkyl und Benzhydryl,
- ein substituierter 1,2,3,4-Tetrahydrochinolinrest substituiert ist mit einem oder zwei gleichen oder verschiedenen Substituenten ausgewählt aus der Gruppe bestehend aus 1-4C-Alkyl und Halogen,
- ein substituierter 1,2,3,4-Tetrahydroisochinolinrest am Benzoteil substituiert ist mit einem oder zwei Substituenten ausgewählt aus der Gruppe bestehend aus Hydroxy, 1-4C-Alkoxy und Di-1-4C-alkylamino,
und wobei
R7 Wasserstoff, 1-4C-Alkyl, 1-4C-Alkoxy, Halogen oder Nitro,
R8 Wasserstoff, 1-4C-Alkyl, 1-4C-Alkoxy, Halogen oder Nitro,
und
R9 Wasserstoff oder Trifluormethyl bedeutet, und die Salze dieser Verbindungen.

2. Verbindungen der Formel I, nach Anspruch 1, in denen R3 Chlor, R4 1-4C-Alkyl, A Ethylen oder Propylen und X CH bedeutet und ihre Salze.

3. Verbindungen der Formel I nach Anspruch 1, in denen R4 1-4C-Alkyl, A 2-4C-Alkylen und R5 1-4C-Alkyl oder Ar-1-4C-alkyl und R6 Ar-1-4C-alkyl bedeutet, oder worin R5 und R6 gemeinsam und unter Einschluß des Stickstoffatoms, an das beide gebunden sind, einen unsubstituierten oder substituierten 6-Ring-Heterocyclus darstellen, der ausgewählt ist aus der Gruppe bestehend aus Piperidin, Piperazin, 1,2,3,4-Tetrahydrochinolin und 1,2,3,4-Tetrahydroisochinolin und die Salze dieser Verbindungen.

4. Verbindungen der Formel I nach Anspruch 1, in denen R3 Chlor, R4 1-4C-Alkyl, A Ethylen oder Propylen und X CH bedeutet, und worin R5 1-4C-Alkyl oder Benzyl und R6 Ar-1-4C-alkyl bedeutet, wobei Ar Phenyl oder Furyl bedeutet, oder worin R5 und R6 gemeinsam und unter Einschluß des Stickstoffatoms, an das beide gebunden sind, einen unsubstituierten oder substituierten 6-Ring-Heterocyclus darstellen, der ausgewählt ist aus der Gruppe bestehend aus Piperidin, Piperazin und 1,2,3,4-Tetrahydroisochinolin wobei
- ein substituierter Piperidinorest substituiert ist mit einem Substituenten ausgewählt aus der Gruppe bestehend aus Phenyl und Benzyl,
- ein substituierter Piperazinorest in 4-Position substituiert ist mit einem Substituenten ausgewählt aus der Gruppe bestehend aus Phenyl, durch R7, R8 und R9 substituiertes Phenyl und Benzyl und
- ein substituierter 1,2,3,4-Tetrahydroisochinolinrest am Benzoteil substituiert ist mit einem oder zwei 1-4C-Alkoxy-Substituenten, und wobei
R7 Wasserstoff oder 1-4C-Alkoxy,
R8 Wasserstoff und
R9 Wasserstoff bedeutet,
und die Salze dieser Verbindungen.

5. Verfahren zur Herstellung der Verbindungen der Formel I nach Anspruch 1, worin R1, R2, R3, R4, R5, R6, X und A die in Anspruch 1 angegebenen Bedeutungen haben, und ihrer Salze, **dadurch gekennzeichnet, daß** man
a) Mercaptobenzimidazole der Formel II 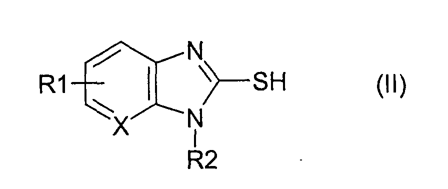 worin R1, R2 und X die in Anspruch 1 angegebenen Bedeutungen haben, mit Picolinderivaten III 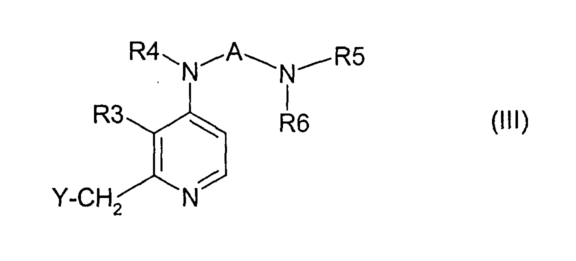 worin R3, R4, R5, R6 und A die in Anspruch 1 angegebenen Bedeutungen haben und Y eine geeignete Abgangsgruppe darstellt, umsetzt oder daß man
b) Verbindungen der Formel IV 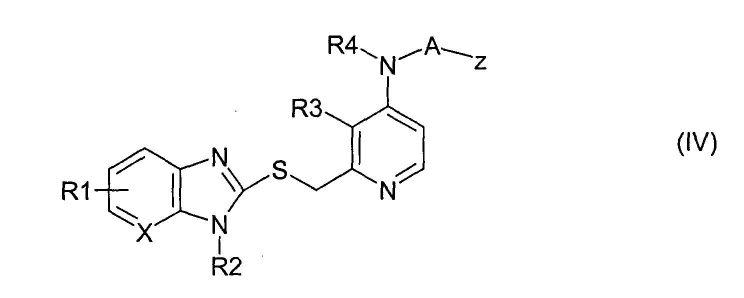 worin R1, R2, R3, R4, X und A die in Anspruch 1 angegebenen Bedeutungen haben und Z eine geeignete Abgangsgruppe darstellt, mit Aminen H-N(R5)R6 umsetzt und daß man erhaltene Verbindungen gewünschtenfalls anschließend in die Salze überführt und/oder daß man erhaltene Salze gewünschtenfalls anschließend in die freien Verbindungen überführt.

6. Arzneimittel enthaltend eine oder mehrere Verbindungen der Formel I nach Anspruch 1 und/oder ihre pharmakologisch verträglichen Salze.

7. Verbindungen der Formel I nach Anspruch 1 und/oder ihre pharmakologisch verträglichen Salze zur Anwendung bei der Bekämpfung von Helicobacter-Bakterien.

8. Verbindungen der Formel I nach Anspruch 1 und/oder ihre pharmakologisch verträglichen Salze zur Anwendung bei der Behandlung und/oder Prophylaxe von Erkrankungen des Magens und/oder Darms.

## Claims

1. Compounds of the formula I 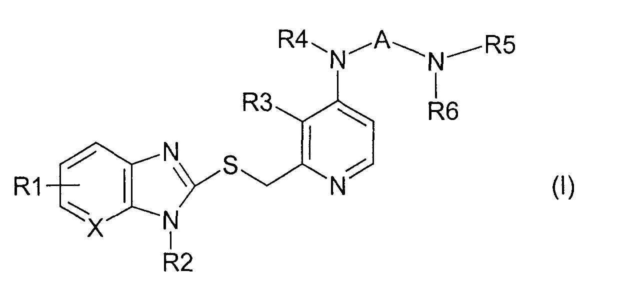 in which
R1 is hydrogen,
R2 is hydrogen,
R3 is halogen,
R4 is 1-7C-alkyl,
A is 1-7C-alkylene and
X is N or CH,
and in which
R5 is 1-7C-alkyl, 3-8C-cycloalkyl or Ar-1-4C-alkyl and
R6 is 1-7C-alkyl, 3-8C-cycloalkyl or Ar-1-4C-alkyl,
where
Ar is phenyl, furyl or phenyl which is substituted by R7, R8 and R9,
or in which
R5 and R6 together represent, with inclusion of the nitrogen atom to which both are bonded, an unsubstituted or substituted 6-membered heterocyclic ring which is selected from the group consisting of piperidine, piperazine, 1,2,3,4-tetrahydroquinoline and 1,2,3,4-tetrahydroisoquinoline,
where
- a substituted piperidino radical is substituted by one or two identical or different substituents selected from the group consisting of 1-4C-alkyl, 1-4C-alkoxy, phenyl, phenyl substituted by R7, R8 and R9, and phenyl-1-4C-alkyl,
- a substituted piperazino radical is substituted in position 4 by a substituent selected from the group consisting of 1-4C-alkyl, 1-4C-alkoxycarbonyl, phenyl, phenyl substituted by R7, R8 and R9, phenyl-1-4C-alkyl, phenyl-1-4C-alkyl substituted by R7, R8 and R9 in the phenyl radical and benzhydryl,
- a substituted 1,2,3,4-tetrahydroquinoline radical is substituted by one or two idenical or different substituents selected from the group consisting of 1-4C-alkyl and halogen,
- a substituted 1,2,3,4-tetrahydroisoquinoline radical is substituted on the benzo moiety by one or two substituents selected from the group consisting of hydroxyl, 1-4C-alkoxy and di-1-4C-alkylamino, and where
R7 is hydrogen, 1-4C-alkyl, 1-4C-alkoxy, halogen or nitro,
R8 is hydrogen, 1-4C-alkyl, 1-4C-alkoxy, halogen or nitro,
and
R9 is hydrogen or trifluoromethyl, and the salts of these compounds.

2. Compounds of the formula I as claimed in claim 1, in which R3 is chloline, R4 is 1-4C-alkyl, A is ethylene or propylene and X is CH, and their salts.

3. Compounds of the formula I as claimed in claim 1, in which R4 is 1-4C-alkyl, A is 2-4C-alkylene and R5 is 1-4C-alkyl or Ar-1-4C-alkyl and R6 is Ar-1-4C-alkyl, or in which R5 and R6 together represent, with inclusion of the nitrogen atom to which both are bonded, an unsubstituted or substituted 6-membered heterocyclic ring which is selected from the group consisting of piperidine, piperazine, 1,2,3,4-tetrahydroquinoline and 1,2,3,4-tetrahydroisoquinoline, and the salts of these compounds.

4. Compounds of the formula I as claimed in claim 1, in which R3 is chlorine, R4 is 1-4C-alkyl, A is ethylene or propylene and X is CH, and in which R5 is 1-4C-alkyl or benzyl and R6 is Ar-1-4C-alkyl, where Ar is phenyl or furyl, or in which R5 and R6 together represent, with inclusion of the nitrogen atom to which both are bonded, an unsubstituted or substituted 6-membered heterocyclic ring which is selected from the group consisting of piperidine, piperazine and 1,2,3,4-tetrahydroisoquinoline, where
- a substituted piperidino radical is substituted by one substituent selected from the group consisting of phenyl and benzyl,
- a substituted piperazino radical is substituted in position 4 by a substituent selected from the group consisting of phenyl, phenyl substituted by R7, R8 and R9, and benzyl, and
- a substituted 1,2,3,4-tetrahydroisoquinoline radical is substituted on the benzo moiety by one or two 1-4C-alkoxy substituents, and where
R7 is hydrogen or 1-4C-alkoxy,
R8 is hydrogen and
R9 is hydrogen,
and the salts of these compounds.

5. Process for the preparation of the compounds of the formula I as claimed in claim 1, in which R1, R2, R3, R4, R5, R6, X and A have the meanings stated in claim 1, and their salts, which comprises
a) reacting mercaptobenzimidazoles of the formula II 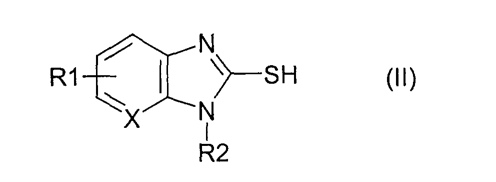 in which R1, R2 and X have the meanings stated in claim 1, with picoline derivatives III 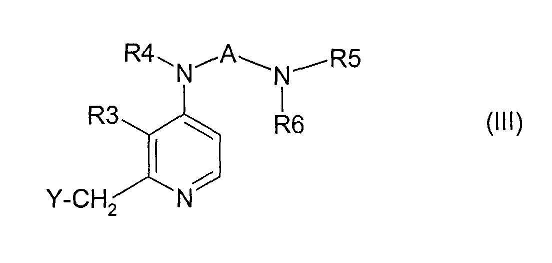 in which R3, R4, R5, R6 and A have the meanings stated in claim 1, and Y is a suitable leaving group, or comprises
b) reacting compounds of the formula IV 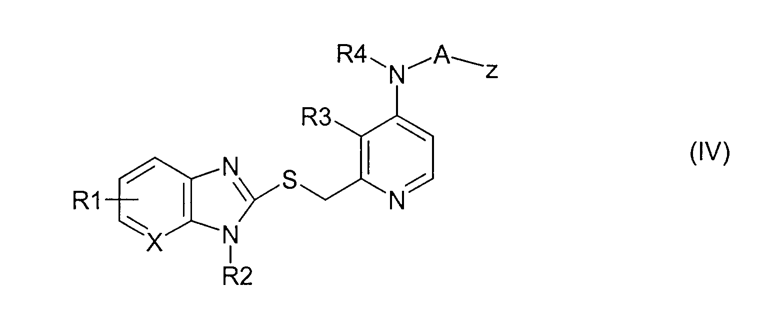 in which R1, R2, R3, R4, X and A have the meanings stated in claim 1, and Z is a suitable leaving group, with an amine H-N(R5)R6 and
comprises subsequently, if required, converting the compounds obtained into the salts and/or comprises subsequently, if required, converting salts obtained into the free compounds.

6. A pharmaceutical comprising one or more compounds of the formula I as claimed in claim 1 and/or their pharmacologically suitable salts.

7. Compounds of the formula I as claimed in claim 1 and/or their pharmacologically suitable salts for use in combating helicobacter bacteria.

8. Compounds of the formula I as claimed in claim 1 and/or their pharmacologically suitable salts for use for the treatment and/or prophylaxis of disorders of the stomach and/or intestine.

## Revendications

1. Composés de formule (I) 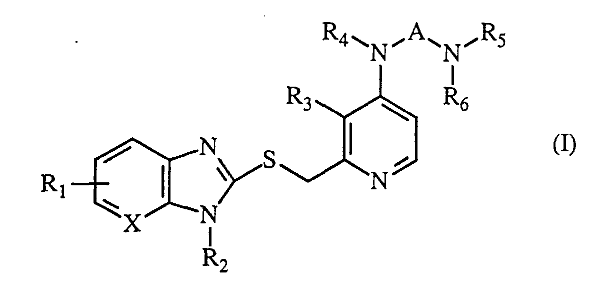 dans laquelle
R₁ représente un atome d'hydrogène,
R₂ représente un atome d'hydrogène,
R₃ représente un atome d'halogène,
R₄ représente un groupe alkyle en C₁₋₇,
A représente un groupe alkylène en C₁₋₇,
X représente N ou CH,
et dans laquelle
R₅ représente un groupe alkyle en C₁₋₇, cycloalkyle en C₃₋₈ ou Ar(alkyle en C₁₋₄) et
R₆ représente un groupe alkyle en C₁₋₇, cycloalkyle en C₃₋₈ ou Ar(alkyle en C₁₋₄), où Ar représente un groupe phényle, furyle ou phényle portant des substituants R₇, R₈ et R₉, ou dans laquelle
R₅ et R₆ représentent conjointement et avec l'atome d'azote auquel ils sont liés, un hétérocycle à 6 chaînons substitué ou non substitué, choisi dans le groupe formé par la pipéridine, la pipérazine, la 1,2,3,4-tétrahydroquinoléine et la 1,2,3,4-tétrahydroisoquinoléine, où
- un résidu pipéridino substitué porte un ou deux substituants, identiques ou différents, choisis dans le groupe formé par alkyle en C₁₋₄, alcoxy en C₁₋₄, phényle, phényle portant des substituants R₇, R₈ et R₉ et phényl-(alkyle en C₁₋₄),
- un résidu pipérazino substitué porte, en position 4, un substituant choisi dans le groupe formé par les résidus alkyle en C₁₋₄, (alcoxy en C₁₋₄)-carbonyle, phényle, phényle portant des substituants R₇, R₈ et R₉, phényl-(alkyle en C₁₋₄), phényl-(alkyle en C₁₋₄) portant sur le noyaux phényle des substituants R₇, R₈ et R₉, et benzhydryle,
- un résidu 1,2,3,4-tétrahydroquinoléine substitué porte un ou deux substituants, identiques ou différents, choisis dans le groupe formé par les groupes alkyle en C₁₋₄ et les atomes d'halogène,
- un résidu 1,2,3,4-tétrahydroisoquinoléine substitué porte, sur le noyau benzène, un ou deux substituants choisis dans le groupe formé par
les résidus hydroxy, alcoxy en C₁₋₄ et di-(alkyle en C₁₋₄)-amino, et où
R₇ représente un atome d'hydrogène, un groupe alkyle en C₁₋₄, alcoxy en C₁₋₄, un atome d'halogène ou un groupe nitro,
R₈ représente un atome d'hydrogène, un groupe alkyle en C₁₋₄, alcoxy en C₁₋₄, un atome d'halogène ou un groupe nitro, et
R₉ représente un atome d'hydrogène ou un groupe trifluorométhyle,
et les sels de ces composés.

2. Composés de formule (I) selon la revendication 1, dans lesquels R₃ représente un atome de chlore, R₄ un groupe alkyle en C₁₋₄, A un groupe éthylène ou propylène et X un groupe CH, et les sels de ces composés.

3. Composés de formule (I) selon la revendication 1, dans lesquels R₄ représente un groupe alkyle en C₁₋₄, A un groupe alkylène en C₂₋₄ et R₅ un groupe alkyle en C₁₋₄ ou Ar(alkyle en C₁₋₄) et R₆ un groupe Ar(alkyle en C₁₋₄), ou dans lesquels R₅ et R₆ représentent conjointement et avec l'atome d'azote auquel ils sont liés, un hétérocycle à 6 chaînons substitué ou non substitué, choisi dans le groupe formé par la pipéridine, la pipérazine, la 1,2,3,4-tétrahydroquinoléine et la 1,2,3,4-tétrahydroisoquinoléine.

4. Composés de formule (I) selon la revendication 1, dans lesquels R₃ est un atome de chlore, R₄ un groupe alkyle en C₁₋₄, A un groupe éthylène ou propylène et X un groupe CH, et dans lesquels R₅ représente un groupe alkyle en C₁₋₄ ou benzyle et R₆ un groupe Ar(alkyle en C₁₋₄) où Ar représente un groupe phényle ou furyle, ou dans laquelle R₅ et R₆ représentent conjointement et avec l'atome d'azote auquel ils sont liés, un hétérocycle à 6 chaînons, substitué ou non substitué, choisi dans le groupe formé par la pipéridine, la pipérazine et la 1,2,3,4-tétrahydroisoquinoléine, où
- un résidu pipéridino substitué porte un substituant choisi dans le groupe formé par les résidu phényle et benzyle,
- un résidu pipérazino substitué porte, en position 4, un substituant choisi dans le groupe formé par les résidus phényle, phényle portant des substituants R₇, R₈ et R₉, et benzyle,
- un résidu 1,2,3,4-tétrahydroisoquinoléine substitué porte, sur le noyau benzène, un ou deux substituants alcoxy en C₁₋₄, et où
R₇ représente un atome d'hydrogène ou un groupe alcoxy en C₁₋₄,
R₈ représente un atome d'hydrogène, et
R₉ représente un atome d'hydrogène, et les sels de ces composés.

5. Procédé pour la fabrication de composés de formule (I) selon la revendication 1, où R₁, R₂, R₃, R₄, R₅, R₆, X et A ont la signification indiquée dans la revendication 1 et de sels de tels composés, **caractérisé par** le fait
a) que l'on fait réagir des mercaptobenzimidazoles de formule (II) 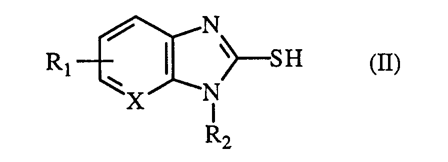 dans laquelle R₁, R₂ et X ont la signification indiquée dans la revendication 1, avec des dérivés de picoline de formule (III) 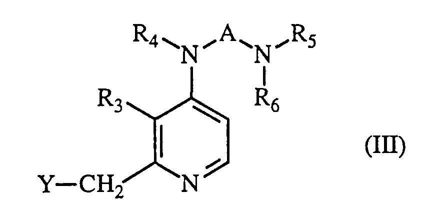 dans laquelle R₃, R₄, R₅, R₆ et a ont la signification indiquée dans la revendication 1, et Y représente un groupe partant approprié, ou
b) que l'on fait réagir des composés de formule (IV) 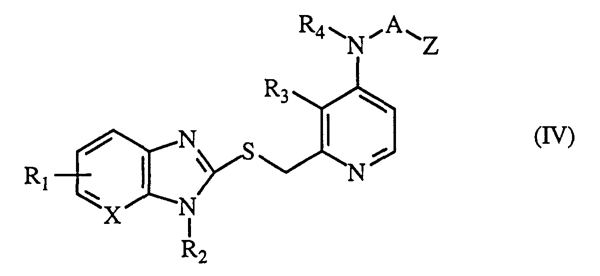 dans laquelle R₁, R₂, R₃, R₄, X et A ont la signification indiquée dans la revendication 1, et Z représente un groupe partant approprié, avec des amines de formule HN(R₅)R₆ et que l'on convertit éventuellement ensuite les composés obtenus en les sels correspondants, et/ou que l'on convertit éventuellement les sels obtenus en les composés libres correspondants.

6. Médicament contenant un ou plusieurs composés de formule (I) selon la revendication 1, et/ou leurs sels pharmacologiquement acceptables.

7. Composés de formule (I) selon la revendication 1 et/ou leurs sels pharmacologiquement acceptables pour lutter contre des bactéries *Helicobacter.*

8. Composés de formule (I) selon la revendication 1 et/ou leurs sels pharmacologiquement acceptables pour le traitement et/ou la prévention de maladies de l'estomac et/ou de l'intestin.
